# EUROPEAN PATENT SPECIFICATION

(11) **EP 2 148 739 B1**
(45) Date of publication and mention of the grant of the patent: **25.02.2015**
(21) Application number: 07748468.1
(22) Date of filing: 07.05.2007
(51) Int. Cl.: B01F 9/06, A61L 2/07, A61L 11/00, B01J 3/04

(54) **APPARATUS FOR TREATING MATERIAL COMPRISING PRESSURE VESSEL WITH DRUM ROTATABLY ARRANGED INSIDE**
VORRICHTUNG ZUR BEHANDLUNG VON MATERIAL MIT EINEM DRUCKGEFÄSS MIT DREHBAR DARIN ANGEORDNETER TROMMEL
APPAREIL DE TRAITEMENT D'UN MATÉRIAU COMPRENANT UNE CUVE SOUS PRESSION ÉQUIPÉE D'UN TAMBOUR ROTATIF INTÉGRÉ

(43) Date of publication of application: 03.02.2010
(73) Proprietor: Sanciflex AB, 148 97 Sorunda (SE)
(72) Inventor: FRÖDERBERG, Per-Arne, 784 36 Borlänge (SE); MADELEY, Melvin, R., Staffordshire WS7 4UH (GB); SEEDHILL, Anna-Lena, Scottsdale, AZ 85251-5116 (US)
(74) Representative: Löfgren, Jonas
(86) International application number: PCT/SE2007/050307
(87) International publication number: WO 2008/136718

(56) References cited:
- EP-A1- 0 575 005
- EP-A1- 0 575 005
- EP-A2- 1 118 706
- WO-A1-00/63483
- WO-A1-2006/015423
- WO-A2-01/26488
- AT-B- 384 009
- DE-A1- 19 922 195
- GB-A- 1 087 550
- US-A- 2 955 305
- US-A- 4 264 741
- US-A- 4 264 741
- US-A- 5 119 994
- US-A- 5 119 994
- US-A- 5 427 650
- US-A- 5 427 650
- US-A- 5 746 987

## Description

### FIELD OF THE INVENTION AND PRIOR ART

The present invention relates to an autoclave apparatus according to the preamble of claim 1, comprising i.a.:
- a pressure vessel having at least one opening for feeding material into the pressure vessel;
- a door for closing said opening of the pressure vessel;
- pressure regulating means for generating a pressure inside the pressure vessel above or below that of the atmosphere; and
- agitation means for agitating material received in the pressure vessel.

The inventive apparatus is particularly intended to be used for treating material in the form of waste by steam at high pressure.

### BACKGROUND ART

It is known to process waste, such as for instance household waste, in an autoclave, in which the waste is subjected to steam at suitable pressure and temperature so as to thereby sterilize the waste and make it safe to handle during the subsequent treatment thereof. The processing of waste in an autoclave will also give other benefits. As an example, organic material within the waste is broken down by the treatment in the autoclave to form a mass of small cellulose particles. Furthermore, plastic objects are reduced in size during the processing in the autoclave and labels and printings on packages of metal, glass and plastic are removed. The mass of cellulose particles may be used for different applications and the remaining waste leaving the autoclave can easily be sorted in order to remove recyclable matter.

A waste treatment autoclave is previously known from WO 2006/056768 A2. This autoclave comprises a pressure vessel which is rotatable about its longitudinal axis in order to agitate waste received inside the pressure vessel. This known autoclave also comprises an internal helix structure projecting from the inner side of the pressure vessel so as to act on waste received in the pressure vessel as the pressure vessel rotates.

EP 0 575 005 A1 discloses an autoclave apparatus according to the preamble of claim 1, for washing and sterilizing rubber plugs or the like, in which a cylindrical basket is rotatably mounted inside a pressure vessel and rotatable by means of a drive unit. Two healical members are mounted inside the cylindrical basket and the apparatus comprises steam ducts for supplying steam into the pressure vessel.

### DISCLOSURE OF THE INVENTION

The object of the present invention is to propose a new and advantageous apparatus for treating material the form of waste, under agitation and at a pressure above or below that of the atmosphere.

According to the invention, this object is achieved by an apparatus having the features defined in claim 1.

The inventive apparatus comprises the features of claim 1.

With the inventive solution, the desired agitation of material treated in the pressure vessel is accomplished in an efficient and simple manner without having to rotate the pressure vessel itself. The pressure vessel may consequently be fixed, which offers several advantages as compared to a rotatably arranged pressure vessel. It is for instance much easier to accomplish a secure and pressure tight clamping of a door to a fixed pressure vessel as compared to a rotary pressure vessel, owing to the fact that the clamping means clamping the door to a fixed pressure vessel do not have to be rotatably arranged. This will facilitate the use of robust and reliable clamping means, which for instance may be hydraulically, pneumatically or electrically actuated. Furthermore, it is easier to mount suitable inlet conduits for steam, compressed air etc in a reliable and pressure tight manner to a fixed pressure vessel as compared to a rotary pressure vessel. The use of a fixed pressure vessel as compared to a rotary pressure vessel will also make it easier to mount suitable heating members to the pressure vessel in order to achieve heating of material received in the pressure vessel.

According to the invention, the drum has an open end projecting through said opening of the pressure vessel, said door being arranged to close the opening of the pressure vessel as well as the opening at this open end of the drum, wherein the door has a part arranged to extend into the opening at said open end of the drum when the door is in its closed position. Owing to the fact that this open end of the drum extends beyond the associated opening of the pressure vessel, material is prevented from falling down into the interspace between the pressure vessel and the drum when being feed into or discharged out of the inner space of the drum.

Further advantageous features of the apparatus according to the invention are indicated in the dependent claims and the following description.

### BRIEF DESCRIPTION OF THE DRAWINGS

With reference to the appended drawings, a specific description of preferred embodiments of the invention cited as examples follows below. In the drawings:
- Fig 1: is a partly cut lateral view of an apparatus according to the present invention, with an inlet arrangement for steam and compressed air shown in a detail enlargement,
- Fig 2: is a schematic cross-sectional view along the line II-II in Fig 1,
- Fig 3: is a schematic cross-sectional view along the line III-III in Fig 1,
- Fig 4: is a schematic cross-sectional view along the line IV-IV in Fig 1,
- Fig 5: is a schematic lateral view of a part of the apparatus of Fig 1 with closed door,
- Fig 6: is a schematic front view of the apparatus of Fig 1 with closed door, and
- Fig 7: is a schematic perspective view of a part of the apparatus of Fig 1 with open door.

### MODES FOR CARRYING OUT THE INVENTION

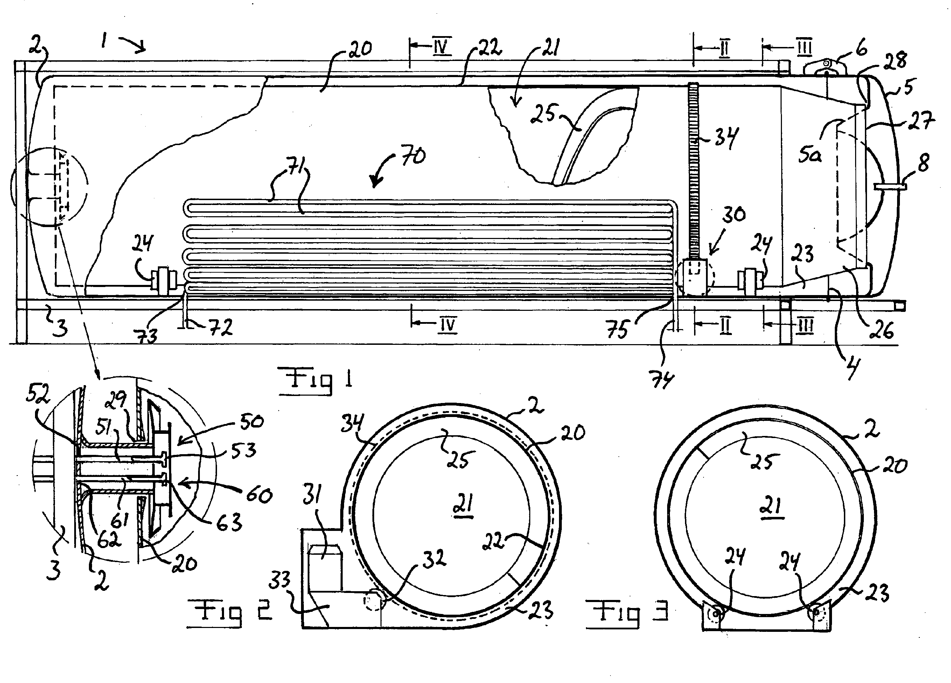

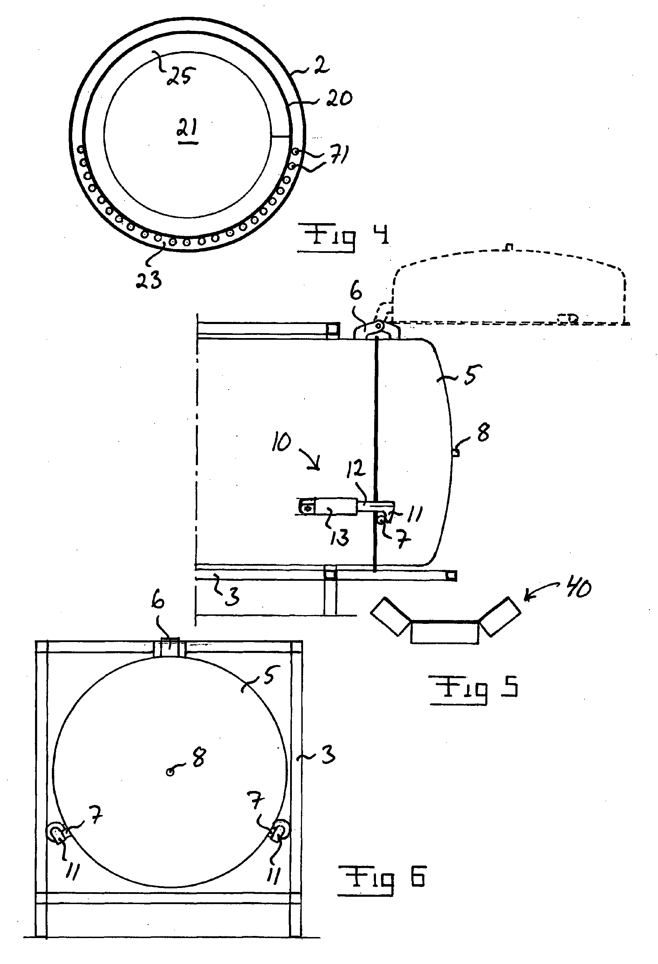

Fig 1 schematically illustrates an apparatus 1 according to the present invention for treating material, for instance material in the form of waste. The apparatus 1 comprises an elongated pressure vessel 2, which is secured to a support structure 3 in the form of a frame, a base or the similar. The pressure vessel 2 has an opening 4 at one end for feeding material into and discharging material out of the pressure vessel. Thus, this opening 4 constitutes an inlet opening for material to be treated in the pressure vessel and also an outlet opening for material treated therein. The opening 4 is closable by means of a pressure tight door 5, which in the illustrated example is hingedly mounted to the pressure vessel 2 via a hinge 6. The apparatus comprises hydraulically, pneumatically or electrically actuated clamping means 10 (see Figs 5-7) for clamping the door 5 tightly to the pressure vessel 2 when the door is in its closed position. The clamping means 10 suitably comprises two or more clamping members 11 distributed about the opening 4. In the illustrated example, the respective clamping member 11 has the form of a hook, which is connected to the piston 12 of a hydraulic cylinder 13 so as to be displaceable between a locking position (see Fig 5), in which the clamping member 11 is in engagement with a pin 7 extending from the door 5 so as to force the rim of the door into pressure tight engagement with the pressure vessel, and an unlocking position, in which the clamping member 11 is out of engagement with the associated pin 7 so as to allow the door 5 to be moved from its closed position (indicated by continuous lines in Fig 5) to its open position (indicated by broken lines in Fig 5). The clamping means 10 could be secured to the pressure vessel 2 or to the support structure 3. The clamping means 10 could of course also be designed in other manners than here illustrated.

As an alternative, the pressure vessel could be provided with an inlet opening at one end for feeding material into the pressure vessel and an outlet opening at the opposite end for discharging material out of the pressure vessel, with a closable door at the respective opening.

The apparatus 1 further comprises agitation means for agitating material received in the pressure vessel 2. The agitation means comprises a drum 20, which is rotatably arranged inside the pressure vessel 2 so as to be rotatable in relation to the pressure vessel. The drum 20 has an inner space 21 for receiving material that is introduced into the pressure vessel via the opening 4 of the pressure vessel. The drum 20 has a cylindrical wall 22 designed to prevent material received in the inner space 21 of the drum from falling into the interspace 23 between the drum 20 and the pressure vessel 2 as the drum rotates in relation to the pressure vessel. The drum 20 is rotatable about its longitudinal axis and is arranged with its longitudinal axis extending in a horizontal or at least essentially horizontal direction. The drum 20 rests on rollers 24, which are rotatably mounted inside the pressure vessel 2, as illustrated in Figs 1 and 3. In the illustrated example, the rollers 24 are idle rollers arranged in pairs at two or more locations along the drum.

The drum 20 is rotated in relation to the pressure vessel 2 by means of suitable drive means 30 (see Figs 1 and 2). In the illustrated example, the drive means 30 comprises a reversible motor 31, for instance a hydraulic motor, arranged inside the pressure vessel 2. The motor 31 drives a rotatably mounted gearwheel 32 via a reduction gear 33. The gear wheel 32 engages with a gear ring 34, which is fixedly secured to the outer side of the drum 20 and surrounds the drum. The gear ring 34 and thereby the drum 20 is rotated in the desired direction when the gear wheel 32 is put into rotation by the motor 31. The drive means 30 could of course also be designed in other manners than here illustrated.

The agitation means further comprises at least one agitation blade 25 mounted along the inner side of the cylindrical wall 22 of the drum so as to act on material received in the inner space 21 of the drum as the drum rotates in relation to the pressure vessel 2. The agitation blade 25 is fixed to the cylindrical wall 22 of the drum so as to rotate together with the drum and it extends in a helical path in the axial direction of said cylindrical wall 22, i.e. in the longitudinal direction of the drum 20, and is suitably a screw-shaped blade. The agitation blade 25 may extend continuously along the cylindrical wall 22 from one end to the other end thereof or be divided into separate parts with intermediate gaps. The agitation blade 25 is arranged to move material received in the inner space 21 of the drum 20 forward away from the opening 4 of the pressure vessel when the drum 20 is rotated by the drive means 30 in a first direction and backward towards said opening 4 when the drum is rotated by the drive means in a second direction opposite said first direction. Thus, the drum 20 is to be rotated in said first direction when material is feed into the inner space 21 of the drum via the opening 4 and in the opposite direction when the material is to be discharged out of the opening 4.

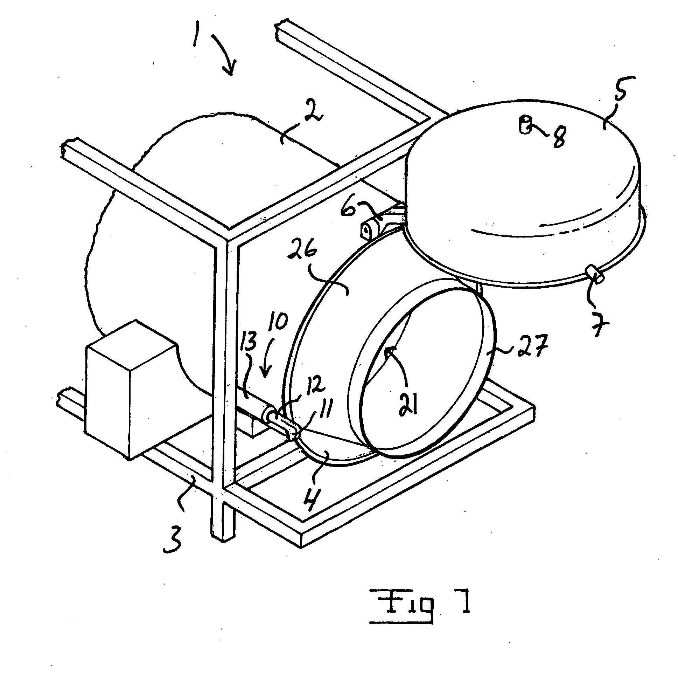

The drum 20 has an open end 26 projecting through the opening 4 of the pressure vessel 2, as illustrated in Figs 1 and 7. Thus, this end 26 of the drum extends beyond the corresponding end of the pressure vessel 2 and material may consequently be feed into and discharged out of the inner space 21 of the drum without falling down onto the bottom of the pressure vessel 2. This end 26 of the drum 20 is suitably tapered towards the opening 27 thereof, as illustrated in Figs 1 and 7. The door 5 is arranged to close the opening 4 of the pressure vessel as well as the opening 27 of the drum and has a part 5a arranged to extend into the opening 27 of the drum when the door is in its closed position. A small gap 28 is suitably provided between the rim of the opening 27 and the door 5 when the door is in its closed position so as to allow the drum 20 to rotate freely in relation to the door.

A conveyor 40, for instance in the form of a belt conveyor, may be arranged under the pressure vessel 2 below the opening 27 of the drum (see Fig 5) so as to receive material discharged out of the drum and carry this material away for further processing.

The apparatus 1 further comprises pressure regulating means 50 for generating a pressure inside the pressure vessel 2 above or below that of the atmosphere. The inner space 21 of the drum 20 is arranged to be in communication with the interspace 23 between the drum and the pressure vessel 2 so as to secure that there is no pressure difference between the inner side and the outer side of the drum. The pressure regulating means 50 may be arranged to generate an overpressure inside the pressure vessel 2 by feeding compressed air and/or compressed steam into the pressure vessel and may be arranged to generate vacuum inside the pressure vessel by exhausting air out of the pressure vessel by suction. In the illustrated example, the pressure regulating means 50 comprises an air supply conduit 51 extending into the inner space 21 of the drum through a pressure tight lead-through 52 at the closed end of the pressure vessel 2 and through a central opening 29 at the inner end of the drum 20, as illustrated in the detail enlargement of Fig 1. An air outlet nozzle 53 is provided in the inner space 21 of the drum at the end of the air supply conduit 51. The pressure regulating means 50 could of course also be designed in other manners than here illustrated.

The apparatus 1 further comprises steam supply means 60 for supplying steam into the inner space 21 of the drum. In the illustrated example, the steam supply means 60 comprises a steam supply conduit 61 extending into the inner space 21 of the drum through a pressure tight lead-through 62 at the closed end of the pressure vessel 2 and through the opening 29 at the inner end of the drum 20, as illustrated in the detail enlargement of Fig 1. A steam outlet nozzle 63 is provided in the inner space 21 of the drum at the end of the steam supply conduit 61. The steam supply means 60 could of course also be designed in other manners than here illustrated. One or several steam outlets is/are arranged in the pressure vessel 2 and/or the door 5. In the illustrated example, the door 5 is provided with such an outlet 8.

The apparatus 1 further comprises heating means 70 for heating material received in the inner space 21 of the drum. The temperature in the inner space 21 of the drum may be controlled by means of the heating means 70 so as to achieve a desired temperature level suitable for the material treatment. In the illustrated example, the heating means 70 comprises heating members 71 (see Figs 1 and 4) in the form of interconnected water conduits extending along a part of the drum 20 in the interspace 23 between the drum and the pressure vessel 2. These heating members 71 are connected to a hot water supply (not shown) via a feeding conduit 72 extending through a pressure tight leadthrough 73 in the pressure vessel and a return conduit 74 extending through a pressure tight lead-through 75 in the pressure vessel. The heating means 70 could of course also be designed in other manners than here illustrated. The heating means could for instance comprise heating members located on the outside of the pressure vessel. The heating means could also comprise electric heating members or any other suitable type of heating members.

The inventive apparatus 1 constitutes an autoclave for treating material in the form of waste.

The invention is of course not in any way restricted to the embodiments described above. On the contrary, many possibilities to modifications thereof will be apparent to a person with ordinary skill in the art without departing from the basic idea of the invention as defined in the appended claims.

## Claims

1. An autoclave apparatus for treating material in the form of waste, the apparatus (1) comprising:
- a pressure vessel (2) having at least one opening (4) for feeding material into the pressure vessel;
- a door (5) for closing said opening (4) of the pressure vessel (2);
- pressure regulating means (50) for generating a pressure inside the pressure vessel (2) above or below that of the atmosphere;
- agitation means for agitating material received in the pressure vessel (2), wherein the agitation means comprises a drum (20), which is rotatably arranged inside the pressure vessel (2) so as to be rotatable in relation to the pressure vessel and which has an inner space (21) for receiving material that is introduced into the pressure vessel via said opening (4) of the pressure vessel, the drum (20) having a cylindrical wall (22) designed to prevent material received in the inner space (21) of the drum from falling into the interspace (23) between the drum (20) and the pressure vessel (2) as the drum rotates in relation to the pressure vessel, and one or several agitation blades (25) mounted along the inner side of said cylindrical wall (22) of the drum so as to act on material received in the inner space (21) of the drum as the drum (20) rotates in relation to the pressure vessel;
- drive means (30) for rotating the drum (20) in relation to the pressure vessel (2); and
- steam supply means (60) for supplying steam into the inner space (21) of the drum (20),
**characterized in:**
- **that** the drum (20) has an open end (26) projecting through said opening (4) of the pressure vessel (2), said door (5) being arranged to close the opening (4) of the pressure vessel as well as the opening (27) at this open end of the drum ; and
- **that** the door (5) has a part (5a) arranged to extend into the opening (27) at said open end (26) of the drum when the door is in its closed position.

2. An apparatus according to claim 1, **characterized in that** the respective agitation blade (25) extends in a helical path in the axial direction of said cylindrical wall (22) of the drum.

3. An apparatus according to claim 2, **characterized in that** the respective agitation blade (25) is a screw-shaped blade.

4. An apparatus according to any of claims 1-3, **characterized in that** the respective agitation blade (25) is arranged to move material received in the inner space (21) of the drum (20) forward away from said opening (4) of the pressure vessel when the drum (20) is rotated by said drive means (30) in a first direction and backward towards said opening (4) of the pressure vessel when the drum (20) is rotated by said drive means (30) in a second direction opposite said first direction.

5. An apparatus according to any of claims 1-4, **characterized in that** the drum (20) is arranged with its longitudinal axis extending in a horizontal or at least essentially horizontal direction.

6. An apparatus according to any of claims 1-5, **characterized in that** the apparatus (1) comprises hydraulically, pneumatically or electrically actuated clamping means (10) for clamping the door (5) tightly to the pressure vessel (2) when the door is in its closed position.

7. An apparatus according to any of claims 1-6, **characterized in that** the apparatus (1) comprises heating means (70) for heating material received in the inner space (21) of the drum.

8. An apparatus according to claim 7, **characterized in that** the heating means (70) comprises one or several heating members (71), for instance in the form of one or several water conduits, arranged in the interspace (23) between the drum (20) and the pressure vessel (2).

9. An apparatus according to any of claims 1-8, **characterized in that** the drum (20) rests on rollers (24), which are rotatably mounted inside the pressure vessel (2).

10. An apparatus according to any of claims 1-9, **characterized in that** the steam supply means (60) comprises a steam supply conduit (61) extending into the inner space (21) of the drum through an opening (29) at the end of the drum facing away from said opening (4) of the pressure vessel.

## Patentansprüche

1. Autoklaviervorrichtung zur Behandlung von Material in der Form von Abfall, wobei die Vorrichtung (1) umfasst:
- ein Druckgefäß (2), das mindestens eine Öffnung (4) zum Hinzugeben von Material in das Druckgefäß aufweist,
- eine Tür (5) zum Schließen der Öffnung (4) des Druckgefäßes (2);
- Druckreguliermittel (50) zum Erzeugen eines Drucks in dem Druckgefäß (2), der über oder unter dem Atmosphärendruck liegt;
- Rührmittel zum Rühren von Material, das im Druckgefäß (2) aufgenommen wird, wobei das Rührmittel eine Trommel (20) umfasst, die drehbar in dem Druckmittel (2) angeordnet ist, um so im Verhältnis zum Druckgefäß drehbar zu sein, und die über einen Innenraum (21) zum Aufnehmen des Materials verfügt, das in das Druckgefäß durch die Öffnung (4) des Druckgefäßes eingeführt wird, wobei die Trommel (20) eine zylindrische Wand (22) aufweist, die gestaltet ist, zu verhindern, dass Material, das im Innenraum (21) der Trommel aufgenommen wird, in den Zwischenraum (23) zwischen der Trommel (20) und dem Druckgefäß (2) fällt, wenn sich die Trommel im Verhältnis zum Druckgefäß dreht, und ein oder mehrere Rührblätter (25), die entlang der Innenseite der zylindrischen Wand (22) der Trommel angebracht sind, um auf Material einzuwirken, das in dem Innenraum (21) der Trommel aufgenommen wird, wenn sich die Trommel (20) im Verhältnis zum Druckgefäß dreht;
- Antriebsmittel (30) zum Drehen der Trommel (20) in Bezug auf das Druckgefäß (2); und
- Dampfzufuhrmittel (60) zum Zuführen von Dampf in den Innenraum (21) der Trommel (20),
- dass die Trommel (20) ein offenes Ende (26) aufweist, das sich durch die Öffnung (4) des Druckgefäßes (2) erstreckt, wobei die Tür (5) angeordnet ist, die Öffnung (4) des Druckgefäßes sowie die Öffnung (27) an diesem offenen Ende der Trommel zu schließen; und
- dass die Tür einen Teil (5a) aufweist, der angeordnet ist, sich in die Öffnung (27) an dem offenen Ende (26) der Trommel zu erstrecken, wenn sich die Tür in ihrer geschlossenen Position befindet.

2. Vorrichtung nach Anspruch I, **dadurch gekennzeichnet, dass** das jeweilige Rührblatt (25) sich in einem spiralförmigen Weg in der axialen Richtung der zylindrischen Wand (22) der Trommel erstreckt.

3. Vorrichtung nach Anspruch 2, **dadurch gekennzeichnet, dass** das jeweilige Rührblatt (25) ein schraubenförmiges Blatt ist.

4. Vorrichtung nach einem der Ansprüche 1 bis 3, **dadurch gekennzeichnet, dass** das jeweilige Rührblatt (25) angeordnet ist, Material, das im Innenraum (21) der Trommel (20) aufgenommen wird, nach vorne fort von der Öffnung (4) des Druckgefäßes zu bewegen, wenn die Trommel (20) durch das Antriebsmittel (30) in eine erste Richtung gedreht wird, und nach hinten zur Öffnung (4) des Druckgefäßes hin zu bewegen, wenn die Trommel (20) durch das Antriebsmittel (30) in eine zweite Richtung entgegengesetzt der ersten Richtung gedreht wird.

5. Vorrichtung nach einem der Ansprüche 1 bis 4, **dadurch gekennzeichnet, dass** die Trommel (20) so angeordnet ist, dass sich ihre Längsachse in eine horizontale oder zumindest im Wesentlichen horizontale Richtung erstreckt.

6. Vorrichtung nach einem der Ansprüche 1 bis 5, **dadurch gekennzeichnet, dass** die Vorrichtung (1) hydraulisch, pneumatisch oder elektrisch betätigte Klemmmittel (10) zum Festklemmen der Tür an das Druckgefäß (2) umfasst, wenn sich die Tür in ihrer geschlossenen Position befindet.

7. Vorrichtung nach einem der Ansprüche 1 bis 6, **dadurch gekennzeichnet, dass** die Vorrichtung (1) Heizmittel (70) zum Erhitzen von Material umfasst, das in dem Innenraum (21) der Trommel aufgenommen wird.

8. Vorrichtung nach Anspruch 7, **dadurch gekennzeichnet, dass** das Heizmittel (70) ein oder mehrere Heizelemente (71) umfasst, zum Beispiel in der Form einer oder mehrerer Wasserleitungen, die im Zwischenraum (23) zwischen der Trommel (20) und dem Druckgefäß (2) angeordnet sind.

9. Vorrichtung nach einem der Ansprüche 1 bis 8, **dadurch gekennzeichnet, dass** die Trommel (20) auf Rollen (24) ruht, die drehbar in dem Druckgefäß (2) gelagert sind.

10. Vorrichtung nach einem der Ansprüche 1 bis 9, **dadurch gekennzeichnet, dass** das Dampfzufuhrmittel (60) eine Dampfzufuhrleitung (61) umfasst, die sich in den Innenraum (21) der Trommel durch eine Öffnung (29) an dem Ende der Trommel erstreckt, das von der Öffnung (4) des Druckgefäßes abgekehrt ist.

## Revendications

1. Appareil autoclave pour le traitement d'un matériau sous forme de déchet, l'appareil (1) comprenant :
- une cuve pressurisée (2) ayant au moins une ouverture (4) pour amener le matériau dans la cuve pressurisée,
- une porte (5) pour fermer la dite ouverture (4) de la cuve pressurisée (2),
- un moyen de régulation (50) de la pression pour générer une pression à l'intérieur de la cuve pressurisée (2) supérieure ou inférieure à la pression atmosphérique,
- un moyen d'agitation pour agiter le matériau reçu dans la cuve pressurisée (2), où le moyen d'agitation comprend un tambour (20) qui est placé de manière rotative à l'intérieur de la cuve pressurisée (2) de façon à pourvoir pivoter par rapport à la cuve pressurisée et qui comporte un espace interne (21) pour recevoir le matériau qui est introduit dans la cuve pressurisée via la dite ouverture (4) de la cuve pressurisée, le tambour (20) ayant une paroi cylindrique (22) conçue pour empêcher le matériau reçu dans l'espace inférieur (21) du tambour de tomber dans l'interstice (23) entre le tambour (20) et la cuve pressurisée (2) quand le tambour pivote par rapport à la cuve pressurisée, et une ou plusieurs pales d'agitation (25) montées le long de la face interne de la dite paroi cylindrique (22) du tambour de manière à agir sur le matériau reçu dans l'espace intérieur (21) du tambour quand le tambour (20) pivote par rapport à la cuve pressurisée,
- un moyen d'entraînement (30) pour faire tourner le tambour (20) par rapport à la cuve pressurisée (2), et
- un moyen d'alimentation en vapeur (60) pour alimenter en vapeur l'espace inférieur (21) du tambour (20), **caractérisé en ce que** :
- le tambour (20) comporte une extrémité ouverte (26) dépassant de la dite ouverture (4) de la cuve pressurisée (2), la dite porte (5) étant agence pour fermer l'ouverture (4) de la cuve pressurisée ainsi que de l'ouverture (27) au niveau de cette extrémité ouverte du tambour, et
- la porte (5) est munie d'une pièce (5a) placée de manière à dépasser à l'intérieur de l'ouverture (27) au niveau de la dite extrémité ouverte (26) du tambour quand la porte est en position fermée.

2. Appareil selon la revendication 1, **caractérisé en ce que** la pale d'agitation respective (25) s'étend selon un trajet hélicoïdal dans la direction axiale de la dite paroi cylindrique (22) du tambour.

3. Appareil selon la revendication 2, **caractérisé en ce que** la pale d'agitation respective (25) est une pale en forme de vis.

4. Appareil selon l'une quelconque des revendication 1 à 3, **caractérisé en ce que** la pale d'agitation respective (25) est placée de manière à déplacer le matériau reçu dans l'espace intérieur (21) du tambour (20) vers l'avant et à distance de la dite ouverture (4) de la cuve pressurisée quand le tambour (20) est mis en rotation par le dit moyen d'entraînement (30) dans une première direction et en arrière vers la dite ouverture (4) de la cuve pressurisée quand le tambour (20) est mis en rotation par le dit moyen d'entraînement (30) dans une deuxième direction opposée à la dite première direction.

5. Appareil selon l'une quelconque des revendication 1 à 4, **caractérisé en ce que** le tambour (20) est positionné avec son axe longitudinal s'étendant dans une direction horizontale ou au moins essentiellement horizontale.

6. Appareil selon l'une quelconque des revendications 1 à 5, **caractérisé en ce que** l'appareil (1) comprend un moyen de serrage (10) actionné hydrauliquement, pneumatiquement ou électriquement pour serrer la porte (5) hermétiquement sur la cuve pressurisée (2) quand la porte est en position fermée.

7. Appareil selon l'une quelconque des revendications 1 à 6, **caractérisé en ce que** l'appareil (1) comprend un moyen de chauffage (70) pour chauffer le matériau reçu dans l'espace intérieur (21) du tambour.

8. Appareil selon la revendication 7, **caractérisé en ce que** le moyen de chauffage (70) comprend un ou plusieurs éléments de chauffage (71), par exemple sous la forme de un ou plusieurs conduits d'eau, placés dans l'interstice (23) entre le tambour (20) et la cuve pressurisée (2).

9. Appareil selon l'une quelconque des revendications 1 à 8, **caractérisé en ce que** le tambour (20) repose sur des rouleaux (24) qui sont montés rotatifs à l'intérieur de la cuve pressurisée (2).

10. Appareil selon l'une quelconque des revendications 1 à 9, **caractérisé en ce que** le moyen d'alimentation en vapeur (60) comprend un conduit d'alimentation en vapeur (61) s'étendant à l'intérieur de l'espace intérieur (21) du tambour à travers une ouverture (29) située à l'extrémité du tambour opposée à la dite ouverture (4) de la cuve pressurisée.
